# EUROPEAN PATENT APPLICATION

(11) **EP 4 173 602 A1**
(43) Date of publication of application: **03.05.2023**
(21) Application number: 21832937.3
(22) Date of filing: 30.04.2021
(51) Int. Cl.: A61F 7/00

(54) **FAT-REDUCING TREATMENT APPARATUS AND FREEZING FAT-REDUCING INSTRUMENT**

(30) Priority: 30.06.2020 CN 202010624372
(71) Applicant: Microport Aesthetics Shanghai (Group) Co., Ltd., Shanghai 201203 (CN)
(72) Inventor: LI, Hongyuan, Jiaxing, Zhejiang 314000 (CN); HUANG, Chubo, Jiaxing, Zhejiang 314000 (CN)
(74) Representative: Lavoix
(86) International application number: PCT/CN2021/091766
(87) International publication number: WO 2022/001357

(57) **Abstract**

A fat-reducing treatment device and a freezing fat-reducing instrument are disclosed. The fat-reducing treatment device includes at least two concave cups (100), each concave cup (100) having a trough-shaped body (110) and a connecting end (120). The at least two concave cups (100) are coupled together at the connecting ends (120) such as to rotatable about a first axis (A). The trough-shaped bodies (110) of the at least two concave cups (100) are brought into communication with each other at the connecting ends (120). The first axis (A) is oriented in a first direction, and the trough-shaped bodies (110) extend in a second direction (B) and are open in a third direction. With this arrangement, the trough-shaped bodies (110) can accommodate the placement of a larger abdomen or waist portion of a human body. In this way, the trough-shaped bodies (110) can adapt to waist and abdomen portions with different contours and curvatures and provide a large fat reduction treatment area, resulting in a simpler treatment procedure, a shorter treatment time, higher fat reduction efficiency, improved fat reduction outcomes and enhanced user experiences and satisfaction.

## Description

### TECHNICAL FIELD

The present invention relates to the field of fat reduction technology and, in particular, to a fat-reducing treatment device and a freezing fat-reducing instrument.

### BACKGROUND

With the improvement of living standards, obesity has become a major problem that plagues people in modern society. Obesity not only goes against people', in particular, women's pursuit of beauty, but also may bring some health problems, such as hypertension, heart disease and diabetes. Traditionally, fat reduction relied mainly on surgery, such as tummy tuck, liposuction and other invasive fat reduction approaches. Although traditional fat reduction methods are effective, their application is largely limited by people's fear of surgery, and non-invasive fat reduction is being increasingly favored.

Existing non-invasive fat reduction approaches on the market mainly include low-level laser therapy (LLLT), radio frequency (RF) therapy, high-intensity focused ultrasound (HIFU) therapy and cryolipolysis. Cryolipolysis is a fat reduction method that utilizes the fact that adipocytes will crystallize and undergo necrosis at a temperature below 10 °C as they are sensitive to cold, leading to an inflammatory response. The dead adipocytes will be eventually phagocytosed and metabolized by macrophages. As suggested by clinical data, cryolipolysis shows good therapeutic effects and can provide satisfactory user experiences. Despite its significant advantages over the other non-invasive fat reduction approaches, existing cryolipolysis-based fat reduction products are associated with a number of drawbacks:
1. A small applicator size leads to operational cumbersomeness and low efficiency, especially in applications of waist or abdomen treatment. As such treatment involves a large area to be treated and requires uniform fat reduction, a small applicator has to perform multiple treatment operations on different parts of the area, each lasting for an equal period of time.
2. In applications of waist or abdomen treatment for fat reduction, applicators cannot adapt to different abdomen and waist contours of different individuals, leading to suboptimal treatment effects, low treatment efficiency and subpar user experiences.

Therefore, it is urgently necessary to develop a fat-reducing treatment device and a freezing fat-reducing instrument, which provide a large fat reduction treatment area and adaptability to different abdomen and waist contours, which result in reduced operational cumbersomeness, improved treatment efficiency and better user experiences.

### SUMMARY OF THE INVENTION

It is an objective of the present invention to address the above-described problems with the prior art, i.e., a small applicator size, inadaptability to different contours, low treatment efficiency and unsatisfactory user experiences, by presenting a fat-reducing treatment device and a freezing fat-reducing instrument.

The above objective is attained by a fat-reducing treatment device according to the present invention, which includes at least two concave cups, each having a trough-shaped body and a connecting end, the at least two concave cups coupled together at the connecting ends in such a manner that they are rotatable about a first axis, the trough-shaped bodies of the at least two concave cups brought into communication with each other at the connecting ends, the first axis oriented in a first direction, the trough-shaped bodies extending in a second direction and being open in a third direction.

Optionally, the fat-reducing treatment device may further include a flexible seal sealingly connected in the second direction to internal walls of the two adjacent concave cups.

Optionally, the fat-reducing treatment device may further include pressing blocks configured to be pressed and squeezed against the flexible seal and thereby secure it at the connecting ends.

Optionally, the pressing blocks may match the flexible seal in shape.

Optionally, the fat-reducing treatment device may further include pins, with respective pin holes being provided at the connecting ends, wherein the pins are configured to be inserted along the first axis through the respective pin holes in the two adjacent concave cups.

Optionally, each concave cup may include at least one vacuum hole in communication with a respective one of the trough-shaped bodies at one end and in connection with an external negative pressure source at the other end.

Optionally, each concave cup may further include at least one vacuum groove, which is formed in a wall of the trough-shaped body and communicates with the vacuum hole.

Optionally, the vacuum groove may extend in the second direction.

Optionally, each trough-shaped body may have a curved cross sectional shape and/or a curved longitudinal sectional shape.

The above object is also attained by a freezing fat-reducing instrument according to the present invention, which includes a cold supply assembly and the fat-reducing treatment device as defined above. The cold supply assemblies are disposed along the third direction on back sides of the concave cups in the fat-reducing treatment device.

Optionally, each cold supply assembly may include a cold exchange assembly, the cold exchange assembly including a cold exchange passage and cold circulation ports, the cold circulation ports brought into communication with opposite ends of the cold exchange passage, the cold exchange passage configured for inlet and outlet of a coolant through the cold circulation ports.

Optionally, each cold supply assembly may further include a sealing plate, which seals the cold exchange passage, and on which the cold circulation ports are provided.

Optionally, each cold supply assembly may include a semiconductor cooler.

Optionally, the freezing fat-reducing instrument may further include a negative pressure component connected to the vacuum holes in the fat-reducing treatment device and configured to provide a negative pressure at the vacuum holes.

Optionally, the freezing fat-reducing instrument may further include a cold output assembly, with the cold exchange assemblies being included in the fat-reducing treatment device, the cold output assembly coupled to the cold exchange assemblies and configured to provide the cold exchange assemblies with the coolant that has been cooled.

The present invention provides a fat-reducing treatment device and a freezing fat-reducing instrument. The fat-reducing treatment device includes at least two concave cups, each having a trough-shaped body and a connecting end. The at least two concave cups are coupled together at the connecting ends such as to rotatable about a first axis. The trough-shaped bodies of the at least two concave cups are brought into communication with each other at the connecting ends. The first axis is oriented in a first direction, and the trough-shaped bodies extend in a second direction and are open in a third direction. In this arrangement, through bringing the at least two trough-shaped bodies into communication with each other, the trough-shaped bodies can accommodate the placement of a larger abdomen or waist portion of a human body. As a result, a larger fat reduction treatment area is provided, resulting in a simpler treatment procedure, a shorter treatment time and higher fat reduction efficiency. The rotatability of the at least two trough-shaped bodies about the first axis enables the trough-shaped bodies to adapt to waist and abdomen portions with different contours and curvatures, resulting in greater adaptability of the product, increased treatment efficiency, better treatment effects and enhanced user experiences and satisfaction.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 schematically illustrates a fat-reducing treatment device according to an embodiment of the present invention;
Fig. 2 is a schematic illustration of one concave cup in the fat-reducing treatment device according to an embodiment of the present invention;
Fig. 3 is a schematic illustration of another concave cup in the fat-reducing treatment device according to an embodiment of the present invention;
Fig. 4 is a schematic illustration of a flexible seal according to an embodiment of the present invention;
Fig. 5 is a schematic illustration of a pressing block according to an embodiment of the present invention;
Fig. 6 schematically illustrates how pins engage with respective pin holes according to an embodiment of the present invention;
Fig. 7 is a schematic illustration of one of the pins according to an embodiment of the present invention;
Fig. 8 is an exploded view of a cold supply assembly according to an embodiment of the present invention;
Fig. 9 schematically illustrates an open configuration of the fat-reducing treatment device according to an embodiment of the present invention;
Fig. 10 schematically illustrates another open configuration of the fat-reducing treatment device according to an embodiment of the present invention.
Fig. 11 is a schematic illustration of the fat-reducing treatment device according to an embodiment of the present invention, as viewed from another angle; and
Fig. 12 schematically illustrates a portion of the fat-reducing treatment device according to an embodiment of the present invention.

In these figures:
A-first axis; B-second direction;
100-concave cup; 110-trough-shaped body; 111-peripheral edge; 120-connecting end; 130-vacuum hole; 140-vacuum groove;
200-cold supply assembly; 210-cold exchange assembly; 211-cold exchange passage; 212-cold circulation port; 2121-inlet port; 2122-outlet port; 230-sealing plate; 231-vacuum hole in the sealing plate;
300-flexible seal;
400-pressing block;
500-screw; 510-screws hole;
600-pin; 610-pin hole.

### DETAILED DESCRIPTION

Objectives, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In addition, the structures shown in the figures are usually partial representations of their actual counterparts. In particular, as the figures would have different emphases, they are sometimes drawn to different scales.

As used herein, the singular forms "a", "an" and "the" include plural referents, unless the content clearly dictates otherwise. As used herein, the term "or" is generally employed in the sense of "and/or", unless the content clearly dictates otherwise.

Embodiments of the present invention provide a fat-reducing treatment device and a freezing fat-reducing instrument, essentially characterized by at least two concave cups, which are coupled together so as to be rotatable about a first axis, thus providing a large fat reduction treatment area and adaptability to different waist and abdomen contours. This device is easy to operate and provides high treatment efficiency and satisfactory user experiences.

Embodiments of the present invention provide a fat-reducing treatment device and a freezing fat-reducing instrument. The fat-reducing treatment device includes at least two concave cups, each having a trough-shaped body and a connecting end. The at least two concave cups are coupled together at the connecting ends in such a manner they are rotatable about a first axis. The trough-shaped bodies of the at least two concave cups are brought into communication with each other at the connecting ends. The first axis is oriented in the first direction, and the trough-shaped bodies extend in a second direction and open in a third direction. In this arrangement, through bringing the at least two trough-shaped bodies into communication with each other, the trough-shaped bodies can accommodate the placement of a larger abdomen or waist portion of a human body. As a result, a larger fat reduction treatment area is provided, resulting in a simpler treatment procedure, a shorter treatment time and higher fat reduction efficiency. The rotatability of the at least two trough-shaped bodies about the first axis enables the trough-shaped bodies to adapt to waist and abdomen portions with different contours and curvatures, resulting in greater adaptability of the product, increased treatment efficiency, better treatment effects and enhanced user experiences and satisfaction. The fat-reducing treatment device may further include a flexible seal, which enables the two trough-shaped bodies to together define a sealed cavity and thereby provide an improved cooling effect. Additionally, the fat-reducing treatment device may further pins, with respective pin holes being provided at the connecting ends. The pins may be inserted through the respective pin holes to mitigate pulling and stretching of a portion of the flexible seal around the connecting ends, thus avoiding the flexible seal from being subjected to large tension forces during its deformations, which may destroy the sealing of the concave cups. More preferably, each concave cup may include at least one vacuum hole and at least one vacuum groove. The vacuum holes enable air in a space sealed between the trough-shaped bodies and a human body to be evacuated to create a negative pressure in the space, which allows the trough-shaped bodies to better fit against the human body, resulting in an increase in efficiency of cryolipolysis. The vacuum grooves can facilitate adhesion of the trough-shaped bodies to the human body and the creation of the negative pressure, resulting even better cryolipolysis outcomes.

The following description is set forth with reference to the accompanying drawings.

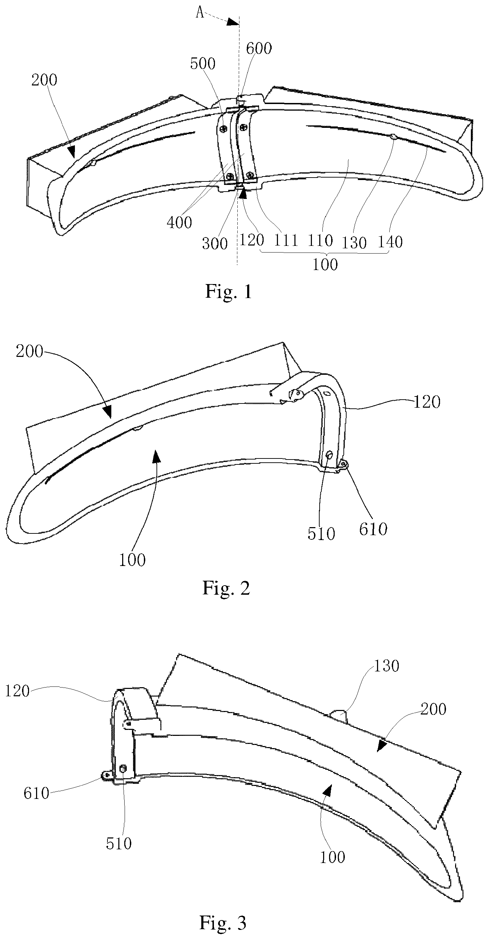

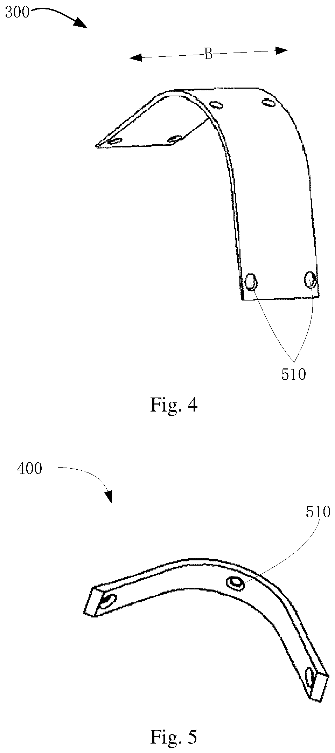

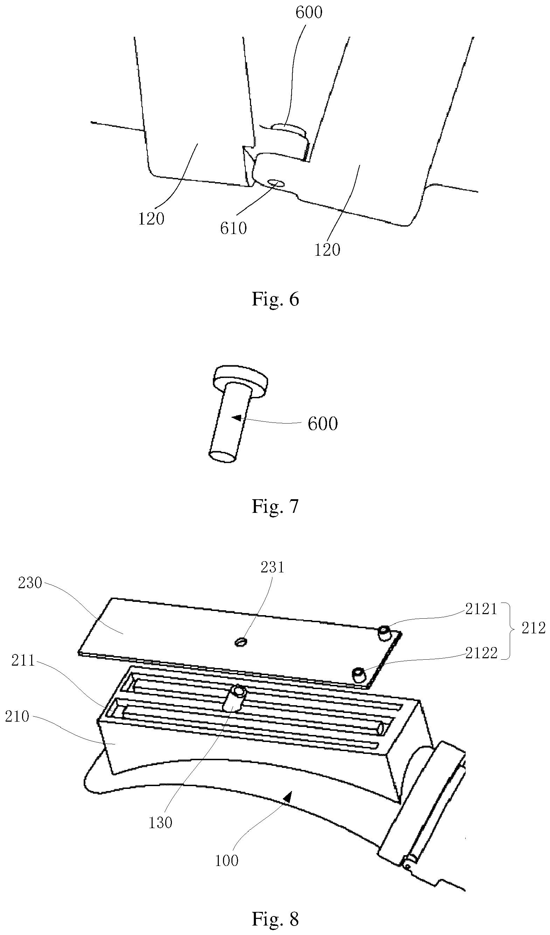

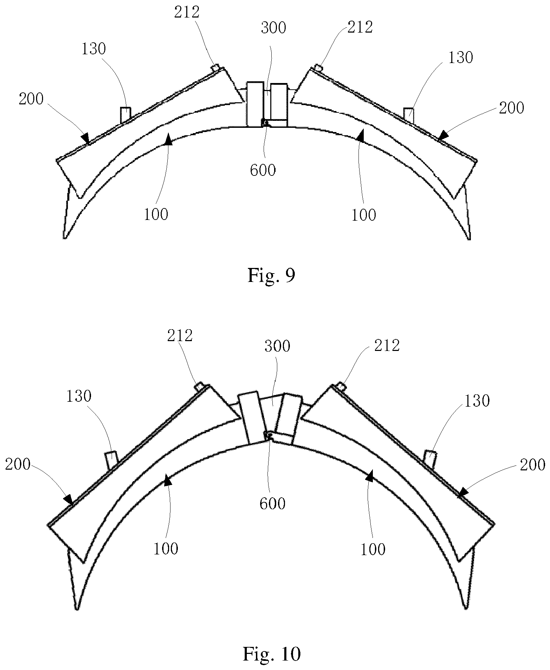

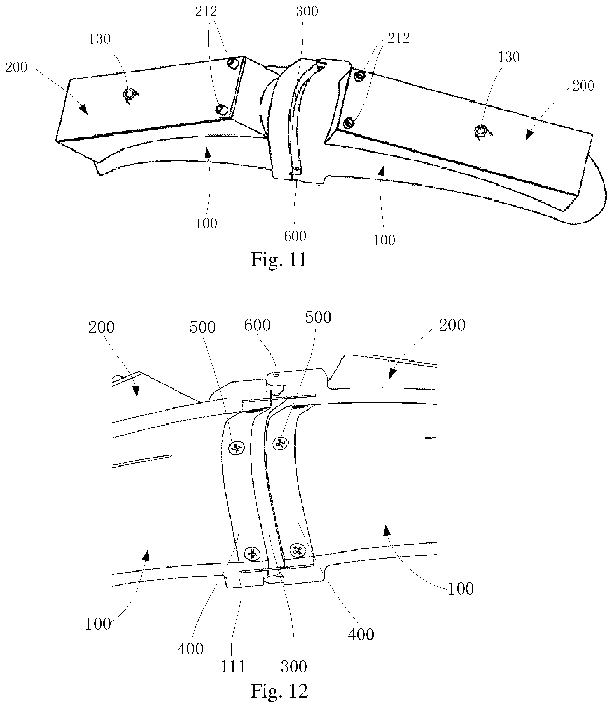

Fig. 1 schematically illustrates a fat-reducing treatment device according to an embodiment of the present invention. Fig. 2 is a schematic illustration of one concave cup in the fat-reducing treatment device according to an embodiment of the present invention. Fig. 3 is a schematic illustration of another concave cup in the fat-reducing treatment device according to an embodiment of the present invention. Fig. 4 is a schematic illustration of a flexible seal according to an embodiment of the present invention. Fig. 5 is a schematic illustration of a pressing block according to an embodiment of the present invention. Fig. 6 schematically illustrates how pins engage with respective pin holes according to an embodiment of the present invention. Fig. 7 is a schematic illustration of one of the pins according to an embodiment of the present invention. Fig. 8 is an exploded view of a cold supply assembly according to an embodiment of the present invention. Fig. 9 schematically illustrates an open configuration of the fat-reducing treatment device according to an embodiment of the present invention. Fig. 10 schematically illustrates another open configuration of the fat-reducing treatment device according to an embodiment of the present invention. Fig. 11 is a schematic illustration of the fat-reducing treatment device according to an embodiment of the present invention, as viewed from another angle. Fig. 12 schematically illustrates a portion of the fat-reducing treatment device according to an embodiment of the present invention.

Referring to Figs. 1 to 5, the fat-reducing treatment device includes at least two concave cups 100.

Referring to Figs. 1 to 3, each of the concave cups 100 includes a trough-shaped body 110 and a connecting end 120.

The trough-shaped bodies 110 extend in a second direction and are open in a third direction. In this way, the trough-shaped bodies 110 can be brought into contact with an abdomen or waist portion of a human body in need of fat reduction. The extension of the trough-shaped bodies 110 in the second direction enables circumferential placement of the concave cups 100 around the abdomen or waist portion of the human body with a larger contact area with the same portion. The trough-shaped bodies 110 are open in the third direction to enable the concave cups 100 to accommodate the placement of the abdomen or waist portion of the human body in the same direction. Each trough-shaped body 110 has, for example, an elongated saddle-like shape with a lengthwise direction extending along the second direction. The saddle-like shape of the trough-shaped body 110 is preferred to have a curved cross section with an inner side corresponding to an interior side of the saddle-like shape and an outer side corresponding to a back side of the saddle-like shape. The interior side faces in the third direction, while the back side faces away from the third direction. The interior side of the saddle-like shape defines a cavity for accommodating an abdomen or waist portion of a human body in need of fat reduction. In addition, a wall of the concave cup 100 at the interior side is defined as an internal wall. Additionally, the saddle-like shape of the trough-shaped body 110 has a longitudinal section with, for example, a curved shape. An inner side of the curved longitudinal section faces in the same direction as the inner side of the curved cross section. Equally, an outer side of the curved longitudinal section faces in the same direction as the outer side of the curved cross section. Thus, the curved longitudinal section can be considered as being open in the third direction. Preferably, the saddle-like shape may have a cross-sectional curvature varying in the second direction. For example, the cross-sectional curvature may be smaller at a farther location in the second direction. The saddle-like shape may have a cross-sectional width varying in the second direction. For example, the cross-sectional width may be smaller at a farther location in the second direction. Of course, those skilled in the art may configure the cross-sectional curvature and width, as well as a longitudinal-sectional curvature and width, as practically required. The trough-shaped body 110 may have an alternative cross-sectional shape, such as a semicircular shape, a wavy shape, a shape consisting of multiple continuous grooves, a trapezoidal shape, or a linear shape. Of course, the cross-sectional shape may also be a combination of the various shapes. For example, it may be a combination of a wavy shape and a curved shape. Alternatively, a curved surface may be added with multiple convexities and concavities, which allow an even larger contact area of the trough-shaped body 110 with the human body portion and hence additionally improved fat reduction outcomes. The trough-shaped body 110 may have an alternative longitudinal-sectional shape, such as wavy or linear.

At the connecting ends 120, for example, a first snap member and a second snap member may be provided. The first snap member may define a recess extending along a first axis A, while the second snap member may define a protrusion extending along the first axis A. The protrusion may fit and snap into the recess, resulting in the at least two concave cups 100 being coupled together at the connecting ends 120 so as to be rotatable about the first axis A. Through coupling the at least two concave cups 100 at the connecting ends 120 in such a manner that they are rotatable about the first axis A, the at least two concave cups 100 can rotate to conform to a curvature of an abdomen or waist portion of a human body. In this way, the at least two concave cups 100 can adapt to various abdomen and waist portions of human bodies with different curvatures. The connecting ends 120 may also be in any other suitable form, as long as the two concave cups 100 are allowed to rotate about the first axis A after they are coupled at the connecting ends 120. The first axis A is oriented in a first direction, the first direction is substantially the same as a widthwise direction of the trough-shaped bodies 110. For example, the widthwise direction of the trough-shaped bodies 110 may be vertical (as in the orientation of Fig. 1). The first direction is a direction from the top of each trough-shaped body 110 to the bottom thereof. It is not necessarily exactly the same as the widthwise direction of the trough-shaped bodies 110, and a certain angle (greater than zero) may be present between the first direction and the widthwise direction of the trough-shaped bodies 110. Once the first direction is determined, the first axis for the at least two concave cups 100 can be determined so as to be oriented in the first direction. In the present embodiment, two concave cups 100 are provided, each concave cup 100 has a connecting end 120. Moreover, the two concave cups 100 are coupled together at the connecting ends 120 such that the concave cups 100 are able to rotate about the first axis A. In alternative embodiments, more concave cups 100 may be provided to provide enhanced adaptability to curvatures. For example, three concave cups 100 (not shown) may be provided, which are connected end to end. In this case, each of the two lateral concave cups 100 may have only one connecting end 120, while the middle concave cup 100 may have connecting ends 120 at both ends. Moreover, the three concave cups 100 may be rotatable about two first axes. It is to be noted that the two first axes may be oriented either both in the first direction, or in different directions. The orientation of each first axis between a corresponding pair of connecting ends may be set or adjusted by those skilled in the art as practically required. In this way, the fat-reducing treatment device can adapt to waist and abdomen portions with different contours and curvatures, resulting in improved treatment efficiency, better treatment effects and enhanced user experiences and satisfaction.

The at least two concave cups 100 are coupled at the connecting ends 120 in such a manner that the trough-shaped bodies 110 of the at least two concave cups 100 are brought into communication with each other at the connecting ends 120. For example, the two saddle-like trough-shaped bodies 110 are joined together at the connecting ends 120 into a single continuous trough. Of course, the two trough-shaped bodies 110 may have different structures, e.g., a combination of two trough-shaped bodies 110 as described above. Bringing the at least two trough-shaped bodies 110 into communication with each other allow the placement of a waist or abdomen portion with a larger area therein for fat reduction. This avoids the problem of multiple repeated operations being required which may arise from the use of a small applicator, making the treatment simpler and more efficient and increasing the product's adaptability.

In one embodiment of the present invention, there is provided a fat-reducing treatment device including at least two concave cups 100, each concave cup 100 has a trough-shaped body 110 and a connecting end 120. The at least two concave cups 100 are coupled together at the connecting ends 120 in such a manner that they are able to rotate about a first axis A. The trough-shaped bodies 110 of the at least two concave cups 100 are brought into communication with each other at the connecting ends 120. The first axis A is oriented in a first direction, and the trough-shaped bodies 110 extend in a second direction and are open in a third direction. In this arrangement, through bringing the at least two trough-shaped bodies into communication with each other, the trough-shaped bodies can accommodate the placement of a larger abdomen or waist portion of a human body. As a result, a larger fat reduction treatment area is provided, resulting in a simpler treatment procedure, a shorter treatment time and higher fat reduction efficiency. The rotatability of the at least two trough-shaped bodies about the first axis enables the trough-shaped bodies to adapt to waist or abdomen portions with different contours and curvatures, resulting in even increased treatment efficiency, better treatment effects and enhanced user experiences and satisfaction.

Preferably, as shown in Figs. 1, 4 and 12, the fat-reducing treatment device includes a flexible seal 300, the flexible seal 300 is sealingly connected in the second direction B to the internal walls of the two adjacent concave cups 100. In this way, the two trough-shaped bodies 110 can together define a sealed cavity to result in an improved cooling effect. In fact, the flexible seal 300 is coupled to the concave cups 100 at the connecting ends 120 thereof. The flexible seal 300 may be directly injection-molded over the trough-shaped bodies 110, or coupled to the trough-shaped bodies 110 by welding or screws. The flexible seal 300 is preferably made of a plastic material. More preferably, the flexible seal 300 is made of an elastomeric material, which enables the trough-shaped bodies 110 to flex at the flexible seal 300 about the first axis A. In this embodiment, the flexible seal 300 is made of silicone rubber, which exhibits good elastic properties and enables an extended service life. Of course, in other embodiments, those skilled in the art can select other materials that can meet the flexibility and other design requirements of practical applications. This present invention is not limited in this regard.

More preferably, in order to additionally ensure that the flexible seal 300 is sealingly connected to the concave cups 100 in a more robust manner, without degradation of the sealing performance when the flexible seal 300 is pulled and stretched during rotation of the trough-shaped bodies 110 about the first axis A, or other undesirable situations, as shown in Figs. 1 and 5, the fat-reducing treatment device may further include pressing blocks 400, the pressing blocks 400 may be made of a metallic or plastic material. More preferably, the pressing blocks 400 may structurally match the flexible seal 300. Specifically, the structure of each pressing block 400 may conform to a widthwise contour of a portion of the trough-shaped body 110 where it is disposed. For example, in this embodiment, as each trough-shaped body 110 is cross-sectionally curved, each pressing block 400 may be curved in the same manner as the widthwise contour of a portion of the trough-shaped body 110 where it is disposed. The interference fit between the pressing blocks 400 and the flexible seal 300 may be pressed against the flexible seal 300 and thereby secure the flexible seal 300 at the connecting ends 120. In other embodiments, the pressing blocks 400 may be a plurality of smaller squares scattered at locations in need of sealing. Through tightly securing the flexible seal 300 to the internal walls of the concave cups 100 by means of the pressing blocks 400, the flexible seal 300 is prevented from being displaced beyond a peripheral edge 111 of any trough-shaped body 110. During a fat reduction treatment, an operator can create a desirably sealed cavity simply by bringing the peripheral edges 111 of the trough-shaped bodies 110 into tight abutment against the skin of a human body. As the pressing blocks 400 prevent the flexible seal 300 from being displaced beyond a peripheral edge 111 of any trough-shaped body 110, any part of the flexible seal 300 will not be located outside the peripheral edges. As a result, when the operator brings the trough-shaped bodies 110 into contact with the human body, the flexible seal 300 will not come into direct contact with the human body. In this way, no gap will be left between the peripheral edges 111 of the trough-shaped bodies 110 and the human body, and an increased sealing effect can be obtained. In embodiments, the pressing blocks 400 may be attached to the flexible seal 300, for example, by injection molding or gluing. Alternatively, the pressing blocks 400 may be integrally formed with the flexible seal 300. In another embodiment, the pressing blocks 400 may be secured to the flexible seal 300 at the connecting ends 120 by screws 500. For example, both the pressing blocks 400 and the flexible seal 300 may have screws holes 510, for insertion of the screws 500 through the screws holes 510.

Further, as shown in Figs. 6 and 7, the fat-reducing treatment device may further include pins 600, and respective pin holes 610 may be provided at the connecting ends 120. The pins 600 may be inserted along the first axis A through the respective pin holes 610 in the two adjacent concave cups 100. The pins 600 may be made of, for example, by a metallic material. The coupling between the pins 600 and the respective pin holes 610 limits freedom of circumferential movement of the concave cups 100 about the first axis A and mitigates pulling and stretching of a portion of the flexible seal 300 around the connecting ends 120. This avoids the flexible seal 300 from being subjected to large tension forces during its deformations and thus prevents the creation of any gap between the flexible seal 300 and the concave cups 100, which may destroy the sealing of the concave cups 100. In alternative embodiments, the pins 600 may also be formed of a plastic material or any other suitable material.

In this embodiment, under the action of the flexible seal 300 and the pins 600, the two concave cups 100 may rotate about the first axis A to form different angles therebetween. For example, Figs. 9 and 10 show different configurations of the two concave cups 100. In the configuration of Fig. 9, as a result of rotation about the first axis A, the two concave cups 100 form therebetween a relatively large angle which allows the fat-reducing treatment device to accommodate an abdomen or waist portion of a human body with a large contour or curvature. In the configuration of Fig. 10, as a result of rotation about the first axis A, the two concave cups 100 form therebetween a relatively small angle which allows the fat-reducing treatment device to accommodate an abdomen or waist portion of a human body with a small contour or curvature.

Preferably, as shown in Figs. 1 and 11, each concave cup 100 may include at least one vacuum hole 130, the vacuum hole 130 communicates with the trough-shaped body 110 at one end and is connected to an external negative pressure source at the other end. The negative pressure source is configured to provide a negative pressure at the vacuum holes 130, which causes air in the sealed space between the trough-shaped bodies 110 and the human body to be evacuated therefrom, leading to a negative pressure in the space. As a result, the trough-shaped bodies 110 can better fit against the human body, resulting in an improvement in efficiency of cryolipolysis. In this embodiment, each concave cup 100 includes one vacuum hole 130 in the shape of a cylindroid. In other embodiments, each concave cup 100 may alternatively include two or more vacuum holes 130. Those skilled in the art may determine the number of vacuum holes 130 and their locations depending on a practical air evacuation need, optionally taking into account the anatomy of the human body. A cross-sectional shape of each vacuum hole 130 may be circular, elliptic, rectangular, trapezoidal, polygonal, like a five-pointed star, with a wavy edge, etc. Each vacuum hole 130 may have either a constant sectional shape or a varying sectional shape across its longitudinal axis.

More preferably, as shown in Fig. 1, each concave cup 100 may further include at least one vacuum groove 140, the vacuum groove 140 is formed in the internal wall of the trough-shaped body 110 and is brought into communication with the vacuum hole 130. The vacuum groove 140 can enhance the efficiency of air evacuation and negative pressure creation and facilitate adhesion of the trough-shaped body 110 to the human body. Each vacuum groove 140 is brought into communication with at least one vacuum hole 130. In this embodiment, each concave cup 100 includes one vacuum groove 140 in communication with one vacuum hole 130. The vacuum groove 140 preferably extends in the second direction, i.e., in the same direction as the trough-shaped body 110 extends, enabling the creation of a more uniform negative pressure as a result of air evacuation. In other embodiments, each concave cup 100 may include two or more vacuum grooves 140, each of which may extend in the trough-shaped body 110 in any direction, e.g., widthwise along a curve cross-sectional contour of the trough-shaped body 110, or at an angle with respect to the second direction. These vacuum grooves 140 may be brought into communication with a single vacuum hole 130. Alternatively, each vacuum groove 140 may be brought into communication with two or more vacuum holes 130. Still alternatively, the vacuum grooves 140 may be brought into communication with respective vacuum holes 130. Other configurations are also possible, without limiting the present invention in any sense. A cross sectional shape of each vacuum groove 140 may be semicircular, semi-elliptic, triangular, rectangular or the like. Each vacuum groove 140 may have either a constant sectional shape or a varying sectional shape across its longitudinal axis.

The present invention further provides a freezing fat-reducing instrument including the fat-reducing treatment device as defined above and cold supply assemblies 200.

The cold supply assemblies 200 are configured to supply cold to the trough-shaped bodies 110, as required by cryolipolysis. Each cold supply assembly 200 is disposed along the third direction on the back side of a respective one of the concave cups 100. The back side is as defined above and, therefore, needs not be described in further detail herein. The freezing fat-reducing instrument retains all the advantages of the fat-reducing treatment device, which have been detailed above and will not be discussed further here. Other components that can be incorporated in the freezing fat-reducing instrument may be those known in the art, and a detailed description of their structure and operation is omitted herein.

As shown in Figs. 1, 8 and 11, each cold supply assembly 200 includes a cold exchange assembly 210, the cold exchange assembly 210 includes a cold exchange passage 211 and cold circulation ports 212 in communication with opposite ends of the cold exchange passage 211. The cold exchange passage 211 is configured for passage of a coolant therethrough via the cold circulation ports 212. The coolant is, for example, a liquid such as a cold (e.g., -20 °C) liquid (e.g., ethylene glycol). The passage of the coolant through the cold exchange passage 211 can bring the cold exchange passage 211 to a target temperature. Those skilled in the art can select different coolants as practically required. The cold exchange passage 211 is, for example, a meandering channel for passage of the coolant therethrough. Those skilled in the art would appreciate that the channel may have a cross section similar to that of a groove. When flowing in the channel, the coolant can transfer cold to the cold exchange passage 211 and further to the trough-shaped body 110, thereby cooling a human body portion in contact therewith for cryolipolysis. In this embodiment, one cold exchange passage 211 in communication with one pair of cold circulation ports 212 for inlet and outlet of the coolant is included. Of course, the cold exchange passage 211 may be otherwise structured, and those skilled in the art may appropriately configure and arrange a proper number of such passages that are shaped and structured in a proper way, as practically need. For example, two cold exchange passages 211 in communication with respective two pairs of cold circulation ports 212 for inlet and outlet of the coolant may be included. The cold circulation ports 212 include, for example, an inlet port 2121 and an outlet port 2122. The coolant can be circulated in each cold exchange passage 211 by entering it through the inlet port 212 and exiting it through the outlet port 2122. The coolant flows into the cold exchange passage 211 at one end and out at the other end. The inlet port 2121 is brought into communication with one end of the cold exchange passage, and the outlet port 2122 with the other end thereof. The cold circulation ports 212 are not limited to being implemented as separate inlet and outlet ports 2121, 2122, and in alternatively embodiments, they may be different ports of a single structure respectively for inlet and outlet of the liquid.

As shown in Figs. 1 and 8, each cold supply assembly 200 further includes a sealing plate 230, the sealing plate 230 is configured to seal the cold exchange passage 211, and the cold circulation ports 220 are provided on the sealing plate 230. In this embodiment, the sealing plate 230 is provided, for example, as a sheet, which seals the cold exchange passage 211 to prevent the coolant from spilling out of the cold exchange passage 211 and ensure that the coolant can flow in the cold exchange passage 211 in an orderly manner. Preferably, the cold circulation ports 220, e.g., inlet and outlet ports 2121, 2122, are provided on the sealing plate 230. In addition, another vacuum hole 231 may be provided in the sealing plate 230, which is brought into communication with the aforementioned vacuum hole 130 to enable it to be connected to the external negative pressure source.

It is to be noted that, as the cold exchange assembly 210 is desired to be kept at a constant temperature during its operation, it is necessary to maintain the coolant at a constant temperature and a constant flow rate and cause it to flow in a uniform manner.

The cold supply assembly 200 may include a semiconductor cooler. In particular, the semiconductor cooler may be a device incorporating a thermoelectric semiconductor material for producing cold. This is based on the phenomenon that when a conductor joined to two different metal blocks is energized with a direct electrical current, one of the joints will be cooled and the other joint will be heated. The semiconductor cooler does not produce noise and vibration, does not need any coolant, operates in a reliable way, is easy to operate and allows adjustability of the amount of cold that it provides.

The freezing fat-reducing instrument may further include a negative pressure component connected to the vacuum holes 130 in the fat-reducing treatment device and configured to provide a negative pressure at the vacuum holes 130. The negative pressure component may be, for example, an air evacuator equipped with air evacuation pipes connected to the vacuum holes 130. The negative pressure component may be a large negative pressure system, for example, equipped with multiple parallel sets of air evacuation pipes. During use, the operator may connect one of the multiple sets of air evacuation pipes to the vacuum holes 130.

More preferably, the freezing fat-reducing instrument further includes a cold output assembly (not shown), and the cold exchange assemblies 210 are included in the fat-reducing treatment device. The cold output assembly is coupled to the cold exchange assemblies 210 and configured to supply the coolant that has been cooled to the cold exchange assemblies 210. The cold output assembly is configured to cool the coolant and may include, for example, a coolant output port, a coolant input port and a pump. The coolant output port is connected to the inlet ports 2121 of the cold exchange assemblies 210, and the coolant input port is connected to the outlet ports 2122 of the cold exchange assemblies 210. The coolant circulates between the cold exchange assemblies 210 and the cold output assembly under the action of the pump.

Below, reference is made to Figs. 1 and 9 to 11 to describe how the freezing fat-reducing instrument according to the present embodiment is used.

First of all, the trough-shaped bodies 110 in the freezing fat-reducing instrument are placed on an abdomen or waist portion of a human body in such a manner that a sealed space is formed between the trough-shaped bodies 110 and the abdomen or waist portion of the human body. If the waist or abdomen portion has a large contour or curvature, the two trough-shaped bodies 110 are rotated about the first axis A to form a larger angle therebetween, which matches the large contour or curvature of the waist or abdomen portion. If the waist or abdomen portion has a small contour or curvature, the two trough-shaped bodies 110 are rotated about the first axis A to form a smaller angle therebetween, which matches the small contour or curvature of the waist or abdomen portion.

Next, a negative pressure is provided at the vacuum holes 130 by the negative pressure component. This may be accomplished by, for example, activating the air evacuator, which then evacuates air from the sealed space between the trough-shaped bodies 110 and the abdomen or waist portion of the human body. As a result, the trough-shaped bodies 110 fit more tightly over the abdomen or waist portion of the human body.

Subsequently, the coolant that has been cooled is provided by the cold output assembly to the cold exchange assemblies 210. The coolant flows in through the inlet ports 2121, transfers cold to the cold exchange passages 211, and flows out through the outlet ports 2122. In this process, as a result of the application of cold, a constant temperature required by the freezing treatment is maintained for a sufficient period of time.

Finally, subsequent to the completion of the freezing treatment, the cold output assembly stops supplying cold and the negative pressure component stops providing the negative pressure. The concave cups 100 are then removed, and the entire treatment process ends.

Of course, the order of operating the various components during the use of the freezing fat-reducing instrument may be properly configured and adjusted by those skilled in the art as practically required.

In summary, the present invention provides a fat-reducing treatment device and a freezing fat-reducing instrument. The fat-reducing treatment device includes at least two concave cups, each having a trough-shaped body and a connecting end. The at least two concave cups are coupled together at the connecting ends such as to rotatable about a first axis. The trough-shaped bodies of the at least two concave cups are brought into communication with each other at the connecting ends. The first axis is oriented in a first direction, and the trough-shaped bodies extend in a second direction and are open in a third direction. In this arrangement, through coupling the at least two trough-shaped bodies together so as to bring them into communication with each other, the trough-shaped bodies can accommodate the placement of a larger abdomen or waist portion of a human body. As a result, a larger fat reduction treatment area is provided, resulting in a simpler treatment procedure, a shorter treatment time and higher fat reduction efficiency. The rotatability of the at least two trough-shaped bodies about the first axis enables the trough-shaped bodies to adapt to waist and abdomen portions with different contours and curvatures, resulting in greater adaptability of the product, increased treatment efficiency, better treatment effects and enhanced user experiences and satisfaction.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A fat-reducing treatment device, comprising at least two concave cups,
each concave cup having a trough-shaped body and a connecting end, the at least two concave cups rotationally connected together about a first axis at the connecting ends, the trough-shaped bodies of the at least two concave cups brought into communication with each other at the connecting ends, the first axis oriented in a first direction, the trough-shaped bodies extending in a second direction and being open in a third direction.

2. The fat-reducing treatment device according to claim 1, further comprising a flexible seal sealingly connected in the second direction to internal walls of two adjacent ones of the concave cups.

3. The fat-reducing treatment device according to claim 2, further comprising pressing blocks configured to be pressed against the flexible seal and thereby secure the flexible seal at the connecting ends.

4. The fat-reducing treatment device according to claim 3, wherein a shape of each pressing block is matched with a shape of the flexible seal.

5. The fat-reducing treatment device according to claim 1, further comprising pins, the connecting ends comprising pin holes, the pins configured to be inserted along the first axis through the respective pin holes in the two adjacent ones of the concave cups.

6. The fat-reducing treatment device according to claim 1, wherein each concave cup comprises at least one vacuum hole, the vacuum hole communicating with a respective one of the trough-shaped bodies at one end and communicating with an external negative pressure source at the other end.

7. The fat-reducing treatment device according to claim 6, wherein each concave cup further comprises at least one vacuum groove, the vacuum groove is formed in a wall of the trough-shaped body and communicates with the vacuum hole.

8. The fat-reducing treatment device according to claim 7, wherein the vacuum groove extends in the second direction.

9. The fat-reducing treatment device according to claim 1, wherein each trough-shaped body has a curved cross sectional shape and/or a curved longitudinal sectional shape.

10. The fat-reducing treatment device according to claim 1, wherein the connecting ends comprise a first snap member and a second snap member, the first snap member defining a recess extending along the first axis, the second snap member defining a protrusion extending along the first axis, the protrusion configured to fit and snap into the recess and thereby couple the at least two concave cups together at the connecting ends in such a manner that the at least two concave cups are able to rotate about the first axis.

11. The fat-reducing treatment device according to claim 1, comprising three concave cups connected sequentially, in which two lateral ones of the concave cups each has one connecting end, and a middle one of the concave cups has two opposite connecting ends, wherein the three concave cups are rotatable about two first axes.

12. A freezing fat-reducing instrument, comprising cold supply assemblies and the fat-reducing treatment device according to any one of claims 1 to 11,
the cold supply assemblies disposed along the third direction on back sides of the concave cups in the fat-reducing treatment device.

13. The freezing fat-reducing instrument according to claim 12, wherein each cold supply assembly comprises a cold exchange assembly, the cold exchange assembly comprising a cold exchange passage and cold circulation ports, the cold circulation ports brought into communication with opposite ends of the cold exchange passage, the cold exchange passage configured for inlet and outlet of a coolant through the cold circulation ports.

14. The freezing fat-reducing instrument according to claim 12, wherein each cold supply assembly further comprises a sealing plate, which seals the cold exchange passage, and wherein the cold circulation ports are provided on the sealing plate.

15. The freezing fat-reducing instrument according to claim 12, wherein each cold supply assembly comprises a semiconductor cooler.

16. The freezing fat-reducing instrument according to claim 12, further comprising a negative pressure component, the fat-reducing treatment device comprising vacuum holes, the negative pressure component connected to the vacuum holes, and the negative pressure component configured to provide a negative pressure at the vacuum holes.

17. The freezing fat-reducing instrument according to claim 12, further comprising a cold output assembly, the fat-reducing treatment device comprising cold exchange assemblies, the cold output assembly connected to the cold exchange assemblies, and the cold output assembly configured to provide the cold exchange assemblies with a coolant that has been cooled.
